(19) Europäisches Patentamt
European Patent Office
Office européen des brevets

(11) **EP 3 735 397 B1**

(12) **EUROPEAN PATENT SPECIFICATION**

(45) Date of publication and mention
of the grant of the patent:
**21.08.2024 Bulletin 2024/34**

(21) Application number: **19702128.0**

(22) Date of filing: **04.01.2019**

(51) International Patent Classification (IPC):
$C02F\ 1/72^{(2023.01)}$   $A61L\ 9/20^{(2006.01)}$
$A61L\ 9/22^{(2006.01)}$   $B01J\ 19/08^{(2006.01)}$
$B01J\ 19/12^{(2006.01)}$   $C01B\ 15/029^{(2006.01)}$
$F24F\ 3/16^{(2021.01)}$

(52) Cooperative Patent Classification (CPC):
**C02F 1/722; C01B 15/027; C01B 15/029**

(86) International application number:
**PCT/IL2019/050025**

(87) International publication number:
**WO 2019/135239 (11.07.2019 Gazette 2019/28)**

(54) **WATER TREATMENT SYSTEM**

**WASSERBEHANDLUNGSSYSTEM**

**SYSTÈME DE TRAITEMENT DE L'EAU**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.01.2018 IL 25674518**

(43) Date of publication of application:
**11.11.2020 Bulletin 2020/46**

(73) Proprietor: **Pollosano Ltd
7611202 Rehovot (IL)**

(72) Inventors:
• **AMAR, Eli
7649008 Rehovot (IL)**
• **SAMIMI GOLAN, Mati
71908 Modieen Macabim Reute (IL)**

(74) Representative: **Harrison IP Limited
3 Ebor House
Millfield Lane
Nether Poppleton
York YO26 6QY (GB)**

(56) References cited:
**US-A- 5 223 105        US-A- 5 622 622
US-A1- 2007 062 883**

## Description

## Technical Field

This invention pertains to non-chemical water cleaning systems and particularly to systems which utilize ambient air transformation into radicalized oxygen gas, which is further generated for water cleaning purposes.

## Background

Water purification has turned an essential requirement due to continuous pollution of water and the need to supply drinkable water. Different methods, chemical and non-chemical, are suggested for water purification. Regarding non-chemical methods, prior art and systems disclosed therein involve only partial aspects of a chamber model for purifying water and related functionalities by producing a purifying reactive gas or air. Accordingly, in all previous prior arts, the integration of the system components comprising UV radiation sources, magnetic field generating sources and air flowing means into the chamber are decoupled as much as possible to obtain the system maximum efficiency and optimal performance. As an example, in US patent No. 4,655,933 to Johnson the ferromagnetic elements, which induce the magnetic flux fields inside the air flowing chamber are located outside or at the corners of the chamber, presumably to eliminate any unwanted perturbation which may be introduced into the ambient air gas which is flowing from inlet to outlet of this chamber, and degrade the whole system performances. As a result, in the related embodiments disclosed in Johnson, the mutual interaction between the ambient air molecules and the magnetic field, induced by the ferromagnetic rods in the disclosed configurations, is limited by the air chamber diameters and by its geometrical shape. These parameters are designed according to different considerations, which include the required air capacity and water cleaning rate. Unfortunately, such design rules and architecture do not leave enough room/degree of freedom for the person skilled in the art to design and make a highly efficient system, which is optimized per the requirements of a certain application and corresponding client needs. Similarly US patent No. 9,321,655 to Kolstad et al disclose similar method and apparatus, however with enhanced performances due to magnetic rods in an anti-symmetric configuration that induce larger magnetic flux on the oxygen gas component of air. Due to the usage of ferromagnetic rods, Johnson and Kolstad suffer from the following problems that degrade the performance of the ionization chamber: i. The magnetic field does not have a coaxial cylindrical symmetry, hence it introduces an non-concentric perturbation to incoming flowing ambient gas. The non-concentric distribution of radicalized gas results in a higher physical interaction between the chamber walls and ambient flowing gas profile which mimics the chamber cylindrical shape. As a result of this perturbation, a higher recombination rate is expected due to higher interaction between the oxygen radicals and ambient gas components, ii. To achieve maximum performance, the magnetic rods and related polarization need to be aligned with respect to the ionization chamber, with respect to other magnetic rods in a given site and between adjacent sites. To overcome this, Kolstad et al embedded the magnetic rods inside long magnetic tubes. The magnetic rods solve the alignment issue however also occupy a significantly high amount of volume inside the ionization chamber lowering its capacity to conduct the compress ambient air. As a result, any minor increase in the rods diameter, which may increase the magnetic field in the chamber, may yield a significant reduction of the free volume of the ionization chamber, limiting the option to optimize the ionization chamber performances.

US 2007/062883 to William describes a device for producing electrically excited oxygen molecules with an array of magnetic rings, pressed against the inner wall of a container and making inner space for accommodating a UV lamp along the length of the container. The magnetic rings populate the entire length of the container and form a continuous magnetic field. As a result, electrically excited air oxygen molecules tend more to recombine with electrically neutral oxygen molecules and fall back to a neutral state.

To compensate the mentioned deficiencies, one can increase UV power specifications or the compressed gas level. However, this can yield in unwanted thermal instabilities and further degrade the ionization chamber performance.

All the required components in the ionization chamber, when correctly configured together may avoid unwanted side effects that can lower the system efficiency degrading its ionization rate and cleaning properties. Such unwanted side effects may be driven by unnecessary increase in the UV power radiation due to scattering and absorption and as a result, unwanted asymmetrical geometrical perturbation that limits the coupling between the UV and ambient gas. Alternatively, the UV power may be enhanced or the rate of compressed air increased. However, any change in the properties of the ionization chamber might modify thermal and other properties of the air flow and as a result degrade system efficiency. In another aspect, an inefficient magnitude of magnetic flux applied on oxygen paramagnetic gas molecules inside the chamber can result in inefficient system, which can function properly only at low compression values of the flowing air.

Moreover, a too high compression gas value or UV power may result in higher gas temperature, significant increase in recombination rate of oxygen gas phase radicals back to their natural diatomic and/or neutral state. This is due to a relatively increased interaction between the oxygen gas molecules and chamber sidewalls and between the radicalized oxygen gas molecules and the other neutral oxygen, nitrogen and other non-radical air molecules.

It is, therefore, an object of the present invention to provide an efficient high performance non-chemical water cleaning system.

## Summary of invention

According to the present invention, there is provided a water purification system as hereafter set forth in Claim 1 of the appended claims.

## Apparatus

The current system injects a compressed ambient air into an inlet of a cylindrical tube shape chamber to produce modified ambient air, transformed through a radicalization process upon exposure to UV light radiation at two different wavelength ranges of UV light 180-195 [nm] and 240-280 [nm]. The ambient oxygen gas component is highly reactive, where its paramagnetic properties are utilized to direct, focus and concentrate it at certain locations using external magnetic flux and magnetically activate it to higher magnetization levels required to enhance excitation process by UV radiation into its radical allotrope phase. The magnetic flux is generated by a certain configuration of concentric ferromagnetic ring shape elements at certain locations inside the chamber. The ambient air, particularly the paramagnetic oxygen molecules which are magnetized by the magnetic field of the rings, is further radiated by the UV light radiation source, which induces its radical higher states of energy. The radicalized oxygen phase comprises an allotrope of several ionized and excited oxygen states and is directed to the tube chamber outlet by external pressure and pumped out into the contaminated water. The water may be stirred, producing the desired kinetics required to improve the solubility of the ionized oxygen radicals, which are pumped into the water. It is assumed that the modified air purifies the water by the formation of hydrogen peroxide ($H_2O_2$) through aggressive reaction of radical oxygen gas molecules, which further react with the contaminants, or alternatively by a direct interaction between the oxygen gas molecule radicals and the contaminations. It is assumed that some part of the oxygen radicals are concentrated in small bubbles which serve as agents that conduct them to direct interaction with water contaminations. The contaminants are either modified or broken into harmless debris which may then be filtered, flushed and drained out of the water containers. The system continuously supplies the modified (active) air in a small bubbles formation to the water to ensure constant purification and supply of purified water.

## Model

Modelling and design of non-chemical water treatment and purification systems such as the one presented in this application is in general a highly complex and non-trivial task which involves various considerations such physical, mechanical and other design considerations. Most of these considerations are derived mainly from several different physical mechanisms which affect directly the performance of the purification system, however also including some mutual interactions between these mechanisms. Hence, in order to yield a highly efficient water treatment and purification system, it is required to correctly employ these physical mechanisms to the ambient air molecules and in particular the paramagnetic oxygen gas molecules, while they flow/propagate through the ionization chamber. Such physical mechanisms and related considerations include the following:

Without limiting the invention to a specific model and embodiments, we assume a general model for the present invention and apparatuses. The general model and methods are applied to apparatus and method which produce oxygen radicals from natural air in a sealed tube with UV radiation, which is further subjected to magnetic field. The model essentially contains several components that need to be considered in the ionization chamber cleaning system. Moreover, in order to achieve a maximum efficiency of the said clearing system, one should consider as well the mutual interactions between the said main components which include:

  i. The geometrical shape and design the ionization chamber and its internal architecture.
  ii. The ambient air flow properties, including its kinetics which is driven by the compression level, paramagnetic properties and thermal properties.
  iii. The magnetic 3D distribution, intensity and flux field.
  iv. The UV radiation field which induces the ambient air gas into radicalized gas phase.

Essentially, the model of the present invention assumes a concentric apparatus with a UV radiation narrow bulb at the center of a cylindrical tube along the entire length of the tube and magnetic round rings made of ferromagnetic material, arranged in pairs around the UV bulb at selected distances. The UV bulb and magnetic rings are held by a central skeleton at a certain stable configuration. The air tube chamber has upper inlet for incoming air and lower outlet for outgoing air exposed to UV radiation and magnetic field. The concentric structure of the apparatus creates a magnetic field that spreads out from the centre of the tube, i.e., the location of the UV lamp/bulb, to the walls of the tube and vertically relative to the centre of the tube. The configuration of the magnetic rings generates a magnetic field that magnetically concentrates, attracting the oxygen molecules in it, due to its paramagnetic properties around a concentric axis at certain geometrical areas in the chamber. As a result of the applied compression pressure, the flux flow direction of the ambient gas in the tube shape ionization chamber is conducted along its longitudinal direction from its inlet to its outlet. The air molecules are mainly drifting, instead of diffusing, along the longitudinal axis of the tube ionization chamber towards the outlet. The local magnetic fields, which are generated by the pairs of rings along the longitudinal axis, temporarily attract oxygen molecules and induce their partial ionization together with the UV radiation. The combination of directional longitudinal transport of the oxygen molecules by the air flux and the transverse and longitudinal direction of stable magnetic fields along the longitudinal axis of the tube yields an efficient time delay of the ambient neutral oxygen gas molecules at the areas with high magnetic flux and further contributes to their conversion to oxygen radicals or excited state oxygen molecules.

Furthermore, the time exposure of the radicalized and/or excited magnetized oxygen molecules in the presence of a magnetic field is proportional to the magnetic field forces and is inversely proportional to the effective kinetic energy of the oxygen molecules, which is derived from the compressed gas level. The paramagnetic oxygen gas molecules are attracted to specific areas of high magnetic flux field and then activated to higher magnetic activation levels and further ionized by the UV radiation bulb. Hence, the design and architecture of the magnetic field, which results in its specific distribution, is a highly important factor in the efficiency of the ionization process. Moreover, the magnetic flux field and the kinetic flux of the air molecules determine the temporary concentration of the radicalized and/or excited oxygen molecules and their ionization rate. The radicalized and/or excited oxygen molecules component is enveloped by the incoming flux of the ambient air. The kinetic interaction between these two components, comprising radicalized and/or excited gas and ambient neutral air and their further interaction and collisions with the ionization chamber walls including its internal skeleton, magnetic rings and UV bulb, drive the recombination rate of the radicalized gas into ambient gas or decay of the excited state. The geometrical shape of the ionization chamber and its internal design are also highly important factors that directly influence its performance and efficiency. As a result, for a given magnetic field architecture, the higher the gas compression level the lesser the magnetic activation enhancement impact on the oxygen gas molecule ionization/excitation rate.

Further, the higher the air flux and the lower the intensity of the magnetic field, the lesser the time of stay of the radical oxygen in the magnetic field, on the one hand, and the lesser the recombination of the radicalized/excited oxygen molecules and return to neutral state on the other hand. The lower the air flux and the higher the intensity of the magnetic field, the greater the time of stay of the oxygen radicals in the magnetic field and the lesser their recombination and decay to neutral state.

In a one embodiment of the present invention, the optimization of the chamber is set according to the following main parameters, which are the geometrical shape and design of the chamber. This design includes its internal architecture comprising the skeleton that carries the magnetic rings and accommodates the UV bulbs and its influence on ambient air flow properties, kinetics which is driven by the compression level, paramagnetic properties and thermal properties, magnetic field distribution, intensity and flux field and UV radiation field, which induces transformation of ambient air into radicalized/excited gas phase. In a further embodiment of the present invention, the dimensions of the magnetic rings are proportional to the dimensions of the chamber. Further, optimization of dimensions of a plurality of rings, internal configuration in a specific magnetic site, which induce the magnetic field intensity distribution and magnetic flux field inside the chamber, is done according to the required radicalization/excitation rate. Also included in evaluation of optimized relative dimensions are the chamber dimensions, geometrical shape, architecture and design, ambient air flow compression level, including kinetics, and paramagnetic and thermal properties and UV radiation field which induces radicalization/excitation of ambient air.

In a further embodiment of the present invention, the magnetic field configuration comprises a plurality of magnetic sites, each site is configured to accommodate one pair of magnetic rings in a similar or opposite magnetic polarity. In a further embodiment of the present invention, the magnetic field induced by the rings in each magnetic site varies from $10^{-3}$ to $10^{+6}$ gauss with sufficient magnetic flux, which is required for a given rate of radicalization/excitation at a certain air compression level and chamber parameters such as geometrical shape and design, internal architecture, ambient air flow properties, including kinetics, air paramagnetic and thermal properties, magnetic field distribution, intensity and flux field and UV radiation field which induces the ambient air into radicalized/excited state. In a further embodiment of the present invention, each magnetic site comprises a pair of magnetic rings with geometrical shape, size, and polarities. In still another embodiment, the contribution of the configuration of the magnetic site to the magnetic field and magnetic field flux in the chamber free volume, including close to its sidewalls, and corresponding contribution in proximity to the magnetic site are considered for inducing radicalization/excitation of air.

In a one preferred embodiment of the present invention, the chamber has a cylindrical shape with two different volumes and lengths of 892 and 430 mm, with similar internal and external diameters of 63.4 mm and 73.15 mm. In still another embodiment, the ferromagnetic rings are made of NdFeB (Grade N42) material coated with Ni-Cu-Ni (Nickel) and have a width of 3.1 mm with external diameter of 31.75 mm, internal diameter of 19.05 mm and thickness of 6.35 mm. In still another embodiment, the diameters of the internal and external pipes at the input and the output of the chamber are 10 mm. The UV lamps have lengths corresponding to the chamber lengths with nominal powers of 21 and 39 watts, respectively. The water reservoir for purification is in volumes in the range of 1000-10000 litters.

In one embodiment of the present invention, the magnetic rings are made of ferromagnetic materials made from rare earth magnets. In particular, the materials are selected from $Nd_2Fe_{14}B$, $SmCo_5$ $Sm_2Co_{17}$, composite magnetic materials such as $BaFe_{12}O_{19}$ , $MnBi$, $Ce(CuCo)5$, a strong permanent magnets such as, Alnico IV/V and Alcomax, which are trade names for composite materials made from alloys of aluminium, nickel and cobalt with iron with additional small amounts of Cu, Ti and Nb and ferrite materials of ferrimanetic materials such as $Fe_2O_3$, and $Fe_3O_4$. In a further embodiment of the present invention, the magnetic field configuration is generated by a plurality of magnetic ring pairs accommodated by a plurality of magnetic sites, wherein each magnetic site comprises one pair of rings comprising one ring made from one of the magnetic materials listed above and one ring made of a metallic material that can be magnetized under induced external magnetic field, such as iron and steel.

The system physical modeling detailed in the previous paragraph may result in various design approaches which can be used for different water purification systems. Such approaches should consider the following main aspects and interactions between the main components of the ionization chamber as follows:

- The air properties include the gas flowing kinetic properties, internal properties inside the ionization chamber, which are affected by the chamber geometrical properties, such as its geometrical shape, dimensions, internal design and materials from which it is made.
- The gas thermal properties such as its temperature and pressure conditions, which are affected by several main factors such as, the external pressure applied on the gas by the compressor, UV heating source/power in addition to all possible other heating sources, which are introduced into the system and may destabilize its thermal properties.
- The gas molecules internal interactions, such as all possible interactions between the ambient radicals including their mutual interactions and interactions with the chamber side walls and all possible obstacles in the chamber while they flow trough it. To avoid a degradation of the system efficiency, it is required to introduce some cooling mechanisms to sustain a stable thermal state with low variations.
- The interaction between the magnetic flux fields and paramagnetic gas molecules induced by the magnetic field, which both magnetically activates and concentrates the paramagnetic oxygen gas molecules into certain areas of high magnetic fields while they flow through the ionization chamber.
- The internal interaction between the paramagnetic oxygen gas molecules, which are activated to higher magnetic energy levels to enhance the ionization probability and rate by the UV radiation.

- The UV radiation source that excites the diatomic oxygen gas molecules into their multivalent allotropic gas phase, comprising several stable states. The UV radiation source is generating hemolytic cleavage of chemical bonds in the air molecules, particularly in the oxygen gas molecules, which flow through the magnetic field resulting in their ionization/excitation and formation of allotropic radicalized/excited gas phase.
- Another aspect is that the oxygen radicals/excited molecules are transported into the water reservoir and their product is, therefore, a variant oxidative reagent that may be expressed as hydrogen peroxide upon reaction with water molecules and different types of oxygen radicals. All types of reagents are reactive oxidants that react with susceptible materials with a potential to be reduced by donating an electron to stabilize the unstable oxygen molecule radicals or reducing the hydrogen peroxide to its stable compounds of water and oxygen.

Considering the previous modeling, we suggest the following embodiments: In one preferred embodiment of the present invention, we propose a fully concentric design for said system comprising a tube shape cylindrical ionization chamber, cylindrical elongated UV radiation bulb and at least one magnetic site comprising of least two magnetic rings which are located symmetrically around the chamber central axis. The magnetic rings are positioned on a skeleton aluminum structure which is designed to hold them in a specific configuration aligning them relative to the ionization chamber central axis, other rings in the specific magnetic site, and other magnetic sites in the ionization chamber. The skeleton structure, including its localized magnetic sites, is designed to minimally perturb the profile and distribution of the incoming flowing ambient and radicalized air components. The magnetic rings can be positioned in parallel, symmetric or anti-parallel, anti-symmetric, magnetic polarization and are configured to induce a maximal concentric magnetic flux field on the compressed air flowing molecules. The magnetic rings radius and shape are defined according to specific requirement of the magnetic flux field, minimizing as well the interaction with the flowing gas. As a result, the radicalized/excited gas profile mimics the magnetic field concentric profile and hence minimally interacts with ambient air flowing components, significantly reducing their mutual interactions and interactions with the chamber walls, internal skeleton and rings. The chamber diameter and length, diameter of the UV radiation bulb and length are selected according to bench mark requirements of required compression level of gas which are predefined by certain required application. These specifications also concern the required operation power and cleaning rate of water of said certain application. After setting these parameters, the magnetic field profile and distribution are set and optimized to achieve the required cleaning in a certain air compression level. As specified, in the current design, the chamber benefits from the concentric design of the magnetic field which significantly reduces the specified interaction of the ionization chamber mentioned above.

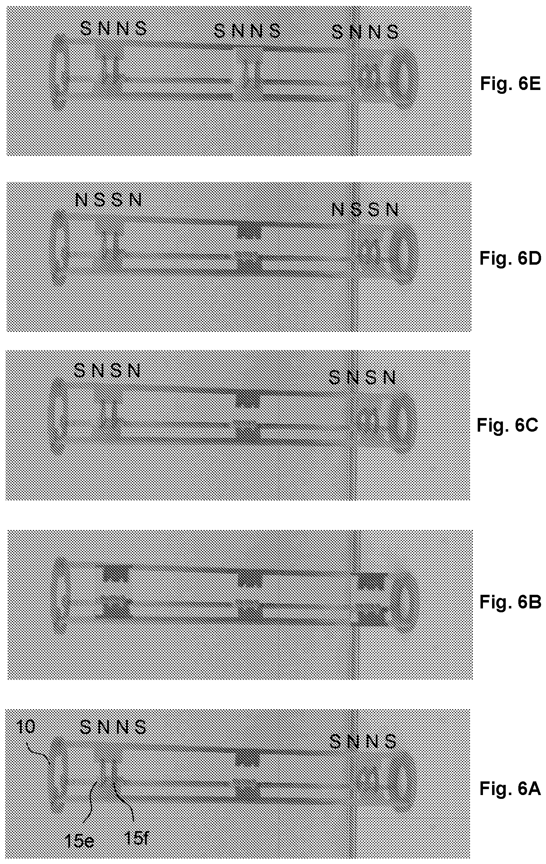

In a further embodiment of the present invention, the ionization chamber design employs a method which utilizes strong interaction between several main physical mechanisms, such as interaction between the oxygen molecules with the magnetic field and UV radiation source while maintaining interaction between gas molecules and other physical mechanisms as low as possible. Such mechanisms may be the air gas flowing profile properties and its thermal properties. This method and design are different from previous prior arts that describe non-chemical water treatment and purification systems, and suggest weak coupling interaction between all the physical mechanisms that control the system. In other words the main objective of this system is to ionize/excite oxygen gas molecules in a most efficient way possible to their radicalized allotropic states, while maintaining as possible normal flow field inside the chamber. Another objective is to suppress the probability and possible recombination mechanisms of the radicalized/excited gas back to diatomic oxygen ground state, while it propagates in the ionization chamber to the air outlet. As a result, we employ a cylindrical symmetric, concentric design of a tube chamber, where both magnetic field and the UV radiation source are located in close proximity to and positioned along the central axis . In addition, as will be further described, the applied magnetic fields comprise two or three sets of concentric cylindrical ferromagnetic rings arranged in the same polarity or opposite polarities according to the configurations in Figs. 6A, C-E, occupying an effective small portion from the total volume of the tube chamber, at the top and bottom ends and center of the tube, where each set of rings comprises negative ring and positive magnetic poles. As a result of this design, it is assumed that the paramagnetic oxygen gas molecules, are concentrated at strong magnetic flux field lines and benefit from a highly strong internal magnetic interaction between with externally applied magnetic field. Additionally, they are in close proximity to the UV radiation ionization source. As a result, the molecules are magnetically activated rather easily into higher magnetization levels with the aid of UV radiation, and are excited to oxygen allotropic phase with a higher generation rate with respect to prior art systems. In addition, due to the highly efficient ionization/excitation rate, the UV radiation source can also operate in rather lower power, introducing a lower average heat magnitude and variations into the oxygen gas molecules that surround it. Hence, it improves its coupling efficiency of the oxygen gas molecules due to elimination of unwanted scattering by neighbor ambient air molecules, such as nitrogen. The compressor pump applied pressure can be lowered and reduce that amount of unwanted components of turbulent flow. In addition, the magnetic chamber geometrical design, including the configuration of ferromagnetic concentric rings, are configured to introduce only minor perturbation into the air flow with minor unwanted turbulent flow side effects. This is achieved with special geometrical design of the tube chamber that takes into account the gas normal flow and ferromagnetic rings and UV bulb/lamp that occupy only a small portion of volume of the chamber. As a result, small perturbations are introduced into the air flow. Further, the ferromagnetic rings induce concentric magnetic field profile along the tube chamber central axis. The radicalized/excited oxygen gas molecules mostly flow close to the tube chamber central longitudinal axis, and experience a small number of recombination events that return them back to their natural oxygen diatomic state. This is due to a relatively small average number of interactions with the chamber sidewalls, easier coupling with the UV radiation and less unwanted scattering events of the UV radiation from the nitrogen gas molecules that degrade the oxygen ionization rate. This is opposed to prior art systems that aim at achieving low coupling between all main physical mechanisms of the system, and accordingly locate at least one of the two main system components, UV lamp and or ferromagnetic elements, externally to the air flow chamber. The system of the present invention employs a cylindrical geometrical configuration for the tube chamber, ferromagnetic rings and UV lamp/bulb, which are located inside the chamber. This maximizes interaction, i.e. radicalization/excitation rate, and minimally perturbs the gas flow profile within the chamber to avoid unwanted turbulent gas flow side effects. It further lowers the recombination process of the radicalized/excited oxygen to diatomic phase, which can degrade the water treatment and purification efficiency. It is believed that the disclosed system benefit from the high/enhanced water purification and treatment efficiency, as demonstrated in the experimental results. As described in the physical modeling section, this benefits from several important contributing factors to yield enhanced water purification and treatment quality.

## Brief Description of the Drawings

The invention will be described further, by way of example, with reference to the accompanying drawings, in which:

> **Fig. 1** shows a schematic illustration of a box diagram of the water purification and treatment system.
> **Fig. 2** shows the internal design of the water purification system.
> **Fig. 3** shows a front view image of the water purification and treatment system.
> Figs. 4A-B show schematic design of the air ionization chamber assembly, where **(A)** shows a top perspective view of the external housing assembly, and **(B)** shows a side perspective view of both internal and external structures and assembly.
> Figs. 5A-D show the design of assembly parts of air ionization chamber. **(A)** shows exploded top perspective view of the external housing assembly parts; **(B)** is an exploded side perspective view of internal and external assembly parts; **(C)** and **(D)** show zoom-in views of **(B)** and **(A)** with and without the ferromagnetic rings, respectively, at the holding seating of the ferromagnetic rings.

Figs. 6A-E show experimented configurations with and without magnetic rings, which are attached to the inner sekeleton inside the ionization chamber.

Figs. 7A-B - (A) show top view images of colour intensity of a DPD (N, N Diethyl-1,4 Phenylenediamine Sulphate) gauge device filled with water from the water container, which is attached to ionization chamber with a certain amount of oxygen radicals. **(B)** shows the corresponding DPD intensity value colour table.

Figs. 8A-B show experimental graph results of radical's concentration **(A)** and steady state time stabilization **(B)** in different air compression flows.

**Fig. 9** shows a graph of the radical concentration measurements in a steady state for different compression flow values.

**Fig. 10** shows graphs of radical concentration experimental results versus different air compression flows multiplied by the corresponding stabilization steady state time.

**Fig. 11** shows a graph of the calculated average oxygen radical flux density for different compression flow values.

## Detailed Description of the Drawings

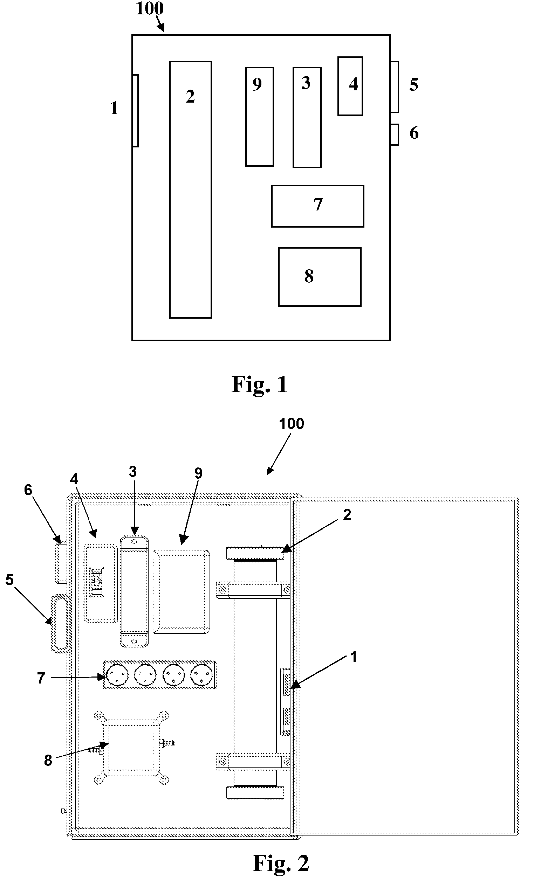

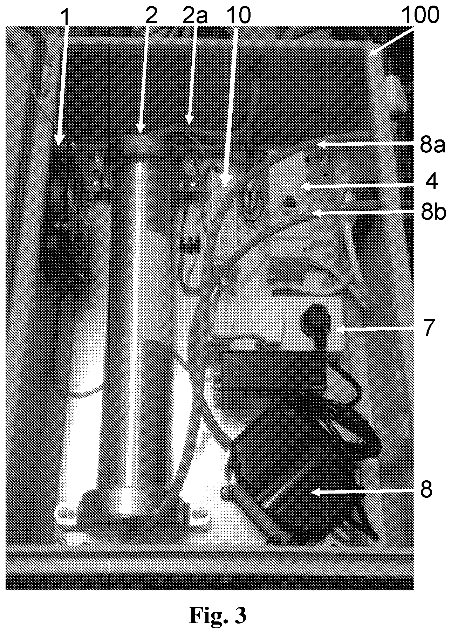

**Figs. 1** and **2** show schematic box diagram and design for water purification and treatment system **(100)**, where a real image of one optional embodiment of the system is shown at **Fig 3.** The water purification system main part comprises: an optional fan cooling system **(1),** which is required to thermally stabilize and regulate the temperature water purification and treatment system as a result of possible unwanted internal or external heating sources. Pending on thermal cooling requirements, the cooling system can employ an air fan, a water cooling or other cooling system; a cylindrical air flow ionization chamber **(2)** made of aluminium, PVC or other chemically inert material, coated with $TiO_2$ on its internal side ; an electrical ballast **(3)** for a UV light bulb/lamp, with specifications of power (Watts, Amps, Volts), connected to the local power supply; an electrical breaker circuit **(4),** added to avoid overloading of the electrical current inside the system; a plurality of gas flow meter devices **(5)** that can be based on electrical or a mechanical flow rate measurement principles, where flow meters can be configured inside or outside the purification system box **(100)** and located anywhere inside or outside the purification and treatment site pending on system requirements. The gas flow meters monitor and regulate the current air gas flow volumetric rate inside the system (measured in values of Litter Per Minute, LPM). A plurality of power meter devices **(6)** are located in any location at the water purification and treatment site and further monitor and regulate the operational values, the system electrical power, voltage and electrical currents. In another embodiment this system is remotely controlled. A plurality of electrical outlets **(7)** enables power supply connections inside and outside the purification and treatment system. The system further comprises compressor air gas **(8).** A regular clean air enters into the compressor or the air is pre-filtered from impurities and contaminations before it enters the ionization chamber with a specific filtering system and is further compressed into the cylindrical tube ionization chamber **(2)** with the air compressor device **(8)** (filtering system not shown in the figure). The compressor pressure values range between 0.1 and 10 [bar] with a flow rate of 2-25 LPM. **Fig. 3** shows one optional setup, in which the air compressor pump **(8)** is connected to gas flow meter devices **(5)** and through it to the ionization chamber with air pipes **(8a, 8b),** respectively. The ionization chamber (2) is connected to the external water reservoir **inlet** (not shown in the related figures) through air gas pipe **(2a).** To improve air intake into the ionization tube chamber, the compressor can be connected to an air diffuser and/or venturi air pipe line. In another embodiment, to improve air flow from the ionization chamber to the water reservoir, the air pipe **(2a)** is replaced with a venturi pipe line that guides it efficiently to contaminated water housing container. In another embodiment of the present invention, the radicalized air flow rate is enhanced by a secondary air compressor or vacuum pump, located at the output pipe **(2a)** at different positions. In such configuration, the secondary air compressor or vacuum pump, push or suck, respectively, the radicalized air toward the diffuser, which is located inside the treated water container or water reservoir. In a further embodiment of the present invention, the air compressor device is connected to the output pipe **(2a)** in proximity to its connection to the ionization chamber outlet. The connection is made with a T-shape air junction element. In this setup, the connection can optionally utilize a non-return air valve connected to the air compressor output and avoid any leak of radicalized air flow or leak into the compressor. The air that flows out of the compressor collides with the radicalized air and accelerates it toward the diffuser which is connected in proximity to its connection to the diffuser device. The connection is done through output pipe **(2a)** outlet, via a T-shape air junction element. A non-return air valve can be connected to avoid leak of radicalized air into the pump. The radicalized air is accelerated by the air pump toward output pipe outlet into the diffuser.

Furthermore, the system comprises a remote control and monitoring unit **(9)** that monitors and controls the system operational values versus their specified ones and can be mechanically or electronically switched between ON and OFF operating states. The monitoring unit monitors the voltage and power supply to the system and particularly voltage and power values of the UV lamp, fan, electronic flow meter and other units in the system.

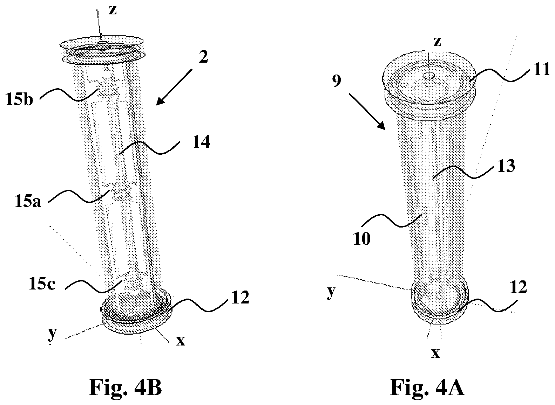

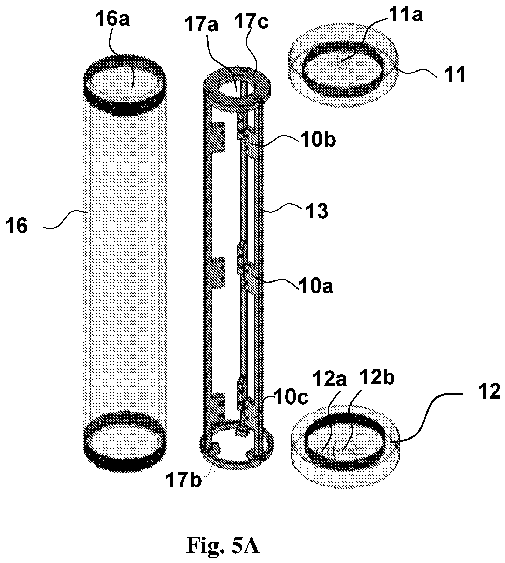

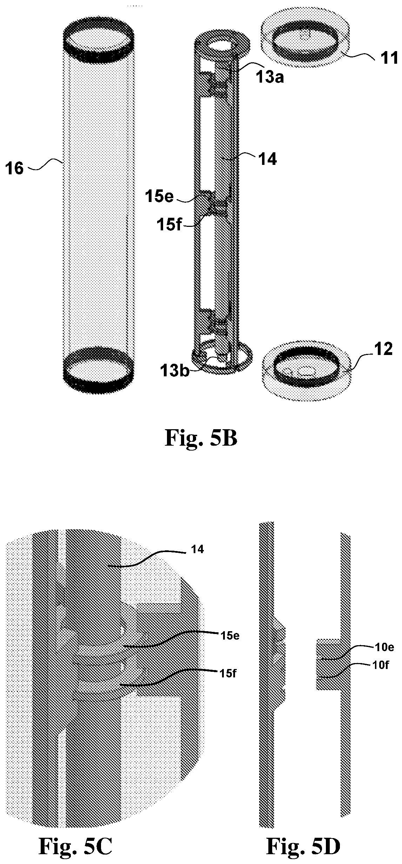

Figs. 4A-B and 5A-D show schematic design of the air ionization chamber in its assembled and unassembled state, respectively. **Fig. 4A** shows a top perspective view of the external housing of the air ionization chamber, where its assembled parts are shown in **Fig. 5A. Fig. 4B** shows a side perspective view of the chamber internal and external structural design, where the assembled parts are shown in **Fig. 5B.** As shown in these figures, the air ionization chamber shown in **Figs. 4A** and **5A,** comprises: A cylindrical housing tube/cylindrical sleeve **(16).** The tube/sleeve may be made of aluminium and PVC (Polyvinyl chloride which is chemically inert) coated on its internal side with $TiO_2$ layer to avoid oxidation and damage by the flowing ambient and radicalized air; A frame/skeleton structure **(13),** with a cylindrical geometrical shape and symmetry. The skeleton may be made of aluminium stainless steel or any hard metal. The skeleton **(13)** is embedded inside the tube/sleeve housing structure **(16).** The frame/skeleton structure is designed with two holding elements **(13a,13b)** for holding the magnetic rings and an internal space for the UV light bulb/lamp **(14).** The skeleton may further comprise holding elements **(10a,10b,10c)** from top to bottom at selected distances from each other for holding ferromagnetic rings in a specific configuration **(15a,15b,15c).** The holding elements or seatings may be made of stainless steel and coated with titanium. The holding elements **(10a, 10b, 10c)** may form a single solid unit with the skeleton. The inner space in the skeleton for the UV lamp is essentially a cage formed by bars along the z-axis and around the centre of the skeleton. The space has openings in proximity to the skeleton bottom and top sides.

The magnetic field configuration comprises three sets of concentric cylindrical ferromagnetic rings **(15a, 15b, 15c)** arranged at selected polarity, occupying an effective small portion of the total volume of the tube chamber. The rings are positioned along the z-axis of the skeleton, particularly at top and bottoms sides and center of the tube chamber main axis, where each set comprises magnetic negative and positive poles rings **(15e, 15f).** In one particular embodiment, the rings are arranged with the same polarity. Generally, the tube and housing are made from chemically and mechanically durable or resistant materials. The UV bulb/lamp **(14)** can comprise two internal lamps that radiate at two wavelength ranges of 180-195 [nm] and 240-280 [nm], and can be designed and produced in two different types and configuration of either mercury filament or LED light. Further, the lamps electrical connector configurations can include 2 or 4 pins and be located at different locations at their sides depending on the light bulb/lamp type. As shown in **Fig. 5C** and **5D,** each of the ferromagnetic ring seating comprises two cylindrical slots **(10e,10f)** configured to mechanically hold two corresponding ferromagnetic rings **(15e,15f).** This design yields a closely packed configuration for the ferromagnetic rings and the UV bulb/lamps **(14)** located along the central longitudinal axis of the air ionization chamber. The ferromagnetic rings are configured to be located close to the UV bulb/lamp radiation source surrounding it at three main locations along the central axis of the air ionization chamber, thus creating three main coupling ionization impact points between the UV radiation and the flowing ambient air Interaction specifically impacts the paramagnetic oxygen component along the ambient air trajectory in the air ionization chamber. The external sleeve structure **(16)** is mechanically attached to top **(11)** and bottom **(12)** covers, disks shaped, made of aluminium or stainless steel materials and further coated by $TiO_2$ layer. The top and bottom covers/caps are configured with one or two holes respectively. The central holes in the top **(11a)** and bottom **(12a)** covers are used as the inlet and outlet for the air flowing through ionization chamber (2), respectively. The bottom housing cover may further be designed with a special second input hole **(12b)** to enable insertion of electrical wiring into and out of the air ionization chamber. In another embodiment, the internal chamber area, including the housing frame **(13),** holding elements and chamber cover internal side are coated with $TiO_2$ to avoid oxidation and damage by the flowing gas inside the chamber.

To enable electrical and vacuum functionalities the inlet and outlet holes are made out of SS (Stainless Steel) resistant material. The covers are mechanically attached to aluminium/SS housing frame **(13)** at its top and bottom bases **(17a, 17b)** and external tube structure **(16).** The external connections of the ionization chamber are sealed with Teflon to ensure the required vacuum condition for air that flows inside the chamber. The attachment to the top and bottom bases **(17a, 17b)** are done with special screws, inserted into holes **(17c)** at the frame top and bottom sides. A plurality of adapter and fastening elements are added to the air and electrical inlets and outlets to enable insertion of electrical input and output lines without affecting internal atmospheric pressure. These elements are also used to enable removal of air from the ionization chamber through specially designed air outlets.

**Example**

In what follows, we have explored the ionization chamber performances and experimental properties and demonstrate its cleaning properties. To this end, we have employed two particular designs and embodiments of the present invention comprising two ionization chambers with two different volumes and lengths of 892 and 430 mm, with similar internal and external diameters of 63.4 mm and 73.15 mm. The ferromagnetic rings made out of NdFeB (Grade N42) material coated by Ni-Cu-Ni (Nickel) with a width of 3.1 mm with external diameter of 31.75 mm, internal diameter of 19.05 mm and thickness of 6.35 mm.

The UV bulbs/lamps had corresponding lengths corresponding to the ionization chamber lengths with nominal powers of 21 and 39 watts, respectively.

To demonstrate the ionization chamber cleaning properties, it was connected to a water reservoir with volume of 1000 litters. For experimental purposes, the ionization chamber with the smaller/higher volume was connected to a small container with volume of 4 litters. The UV radiation lamp was identical in all experiments and demonstrations. The internal and external pipe diameters at the input and the output of the ionization chamber were 10 mm.

Figs. 6A-E show perspective side view images of different configurations of the magnetic rings inside the ionization chamber. The magnetic rings are carried by holding elements **(10)** of the skeleton inside the ionization chamber. As shown in **Fig. 4B,** the magnetic rings are symmetrically aligned relative to the main longitudinal central axis of the holding element **(10)** around the UV bulb **(14)** and the main central axis of the ionization cylindrical chamber. **Fig. 6A** shows a perspective side view image of the anti-symmetric magnetic field configuration comprising two magnetic sites located at two sides of the carrier holding device **(10)** inside the ionization chamber. In this configuration, each of the magnetic site comprises two magnetic rings **(15e, 15f).** The ring polarity is marked as (SN, S = South, N = North), where each ring is positioned in opposite magnetic polarization with its norths pole at its proximal side and South Pole at its distal side, i. e. (SN) (NS). This configuration is marked as the reference configuration in one preferred embodiment of the present invention. **Fig. 6B** shows a perspective side view image of the magnetic field configuration comprising ionization chamber with no magnetic fields. **Fig. 6C** shows the symmetric configuration of the magnetic field in another embodiment of the present invention. The related configuration comprises two magnetic sites, which are located at two sites of the holding element **(10)** inside the ionization chamber. Each magnetic site comprises two magnetic rings **(15e, 15f).** The magnetic rings in each site in this configuration are positioned in the same magnetic polarization direction, which is directed from ionization chamber inlet to its outlet from north to south poles, respectively, i. e. **(NS) (NS). Fig. 6D** shows another optional anti-symmetric magnetic field configuration comprising magnetic rings in another embodiment of the present invention. This configuration comprises two magnetic sites located at the two sides of the holding element **(10)** and ionization chamber. Each site comprises two magnetic rings **(15e, 15f),** which are positioned in opposite magnetic polarization with their south magnetic pole at their proximal sides and north magnetic pole at their distal sides, **(NS) (SN). Fig. 6E** shows the anti-symmetric magnetic field configuration comprising magnetic rings in another preferred embodiment of the present invention. The configuration comprises three magnetic sites located along the central axis and at two sides of the holding element **(10),** as shown in Fig. 4E. In this configuration, each magnetic site comprises two magnetic rings **(15e, 15f),** which are positioned in an opposite magnetic polarization with their norths magnetic pole at their proximal side and south magnetic poles at their distal sides, i.e., **(SN) (NS)** three magnetic sites.

In this measurement, the dissolved DPD at certain density of radicles results in a certain colour and related colour intensity. Experiments performed for ionization chamber with magnetic rings in anti-symmetric reference configuration, shown in **Fig. 6A,** and on ionization chamber without magnetic field shown in **Fig. 6A.** Experiments conducted by a chemical DPD gauge showed inside small water which has been attached to ionization chamber.

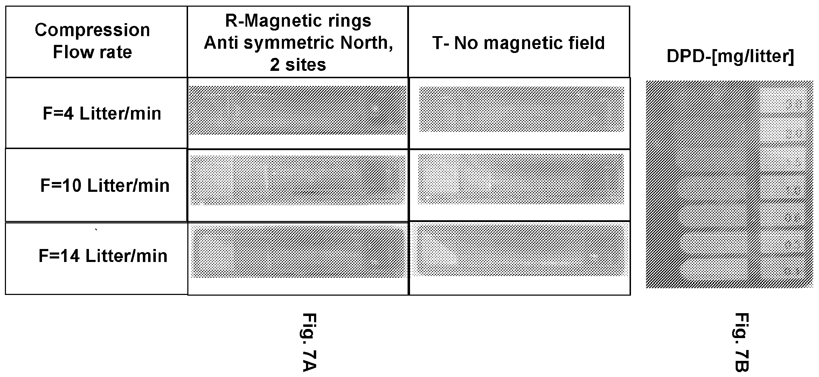

**Fig. 7A** shows top view images of a DPD gauge crucible filled with coloured water which indicates presence of some oxygen radical concentration. The DPD gauge is filled with water and dissolved DPD material and a certain amount of oxygen radicles, which results in a certain colour intensity. The experimental results for the ionization chamber setup, presented in **Figs. 1-5,** reflect experiments done with a small partially closed small size water container with veturi pump and a diffuser at its outlet. The container internal volume was 4 litters which and filled to almost full capacity with a 3.7 litters of water. The experiments were conducted for different air compression flow values of F=4 - 14 litter/min. Furthermore, the experimental results shown in the related table were performed for ionization chamber with magnetic rings in the anti-symmetric reference configuration, **(SN) (NS),** with two magnetic sites, shown in **Fig. 6A,** and ionization chamber without magnetic field, as shown in **Fig. 6B.** The experimental results were arranged in a table according to oxygen gas radical flow value and specific configuration inside the ionization chamber. The ionization chamber **(2)** was turned to ON "state" to generate the oxygenated gas radicals, which flew into the water container trough venturi pump **(2a)** and a diffuser. The water container reaches a steady state after time, **T0.** After the water container reaches a steady state, a chemical DPD measurement is performed. The DPD chemical measurements are performed by inserting the DPD gauge into the water container in a connected vessel configuration. A DPD pill is then inserted into the DPD gauge, quickly dissolved inside the DPD gauge water and performs a chemical reaction with the radicals that enter the gauge top side from the water container. The chemical interaction modifies the colour of the water in the attached test water container. At the end of the chemical reaction between the oxygen radicles and DPD in the water container, the color of the radicalized water is modified from a transparent water regular color to a dark pink color, depending on the radicals concentration inside the gauge device.

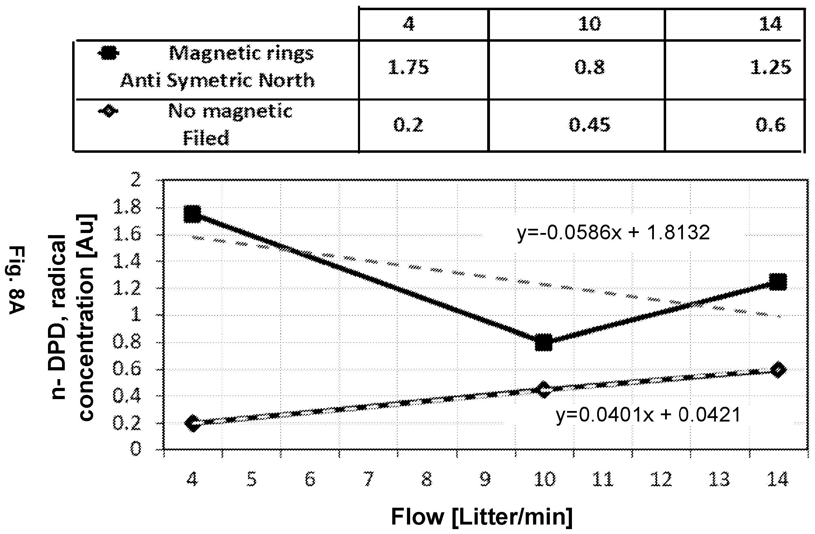

The values of the corresponding intensity are evaluated by using the DPD color intensity table shown in **Fig. 7B.** The specific DPD color-intensity scale is calibrated for ionized chlorine. However, preliminary experiments establish that it is interacts with oxygen radicals and hydrogen peroxide. To use this table experimentally without performing an accurate modelling of the chemical reaction between the radicals and the DPD, we have performed preliminary experiments. In these experiments, we have tested the ionization chamber without magnetic field with and without a UV bulb at different compression flows. In the experiments without an active UV bulb, we found that water color in the container was transparent as the regular water color. In further experiments, we turned the UV bulb on, and set the compression flow to a value of F=4 litter/min. At steady state, we noticed color change that suggested a certain concentration of radicals, see **Fig. 7A** table, second column at top side. By increasing the magnitude values of the compression flow in the range of 4-14 litter/min the color intensity is linearly increased, suggesting higher concentration of free radicals and $H_2O_2$ in the water, see **Fig. 7A,** second column and **Fig. 8A.** This clearly indicates linear correlation trend between the compression flow magnitude and variation in the intensity of the measured color of the water, shown in **Fig. 8A,** for ionization chamber in a configuration without magnetic field. This proves a linear correlation to the radicals concentration which is theoretically expected to be proportional to the compression flow magnitude assuming first order reaction of the radicals with DPD in the water. As a result, we have shown that we can use DPD color intensity table to evaluate the relative level of the radicals concentration at different compression flow values and with different configurations. This is, however without exact quantification of these concentration values. Hence, we have modified the units of the intensity scale from [mg/min] to arbitrary unit ones marked as **[AU].**

Without limiting the invention to the following theoretical discussion, these experiments show that radicalized/excited oxygen gas exits through the ionization chamber outlet assisted by venturi pipe **(2a)** and diffuser (not shown) into the water container. This results in several reactant/product phases comprising: **i.** A main component of free radicals encapsulated inside air bubbles containing various oxygenated allotropic oxygen radicals; **ii.** $H_2O_2$, Hydrogen Peroxide, produced upon chemical reaction between the oxygenated radicalized gas and the water in the container. **iii.** Short life-time free radicals that do not chemically react with water. There might be other types chemical reactants/products which flow out of the ionization chamber into the water container.

Experiments were performed with ionization chamber connected to a small water container, using the DPD experimental gauge setup shown in **Fig. 6,** for ionization chamber with magnetic rings in anti-symmetric reference configuration, shown in **Fig. 6A,** and for ionization chamber without magnetic field shown in **Fig. 6B.**

Without limiting the invention to the following experimental discussion, it has been noticed and hence it is assumed that the main reactants that participate in the cleaning processing of the contaminated water and further chemically interact with DPD are components **i** and **ii.** Furthermore, we found reacting/product component **i** (free radicals which are encapsulated inside air bubbles) to be the most reactive.. The main phase of the radicals are bubbles that diffuse into water in the container, flow up into the air-water interface due to buoyancy forces, thereby creating arrays of bubbles along that interface. In the practical cleaning mode with the ionization chamber, the bubbles that flow through the water reservoir serve as agents that deliver the radicals to direct interaction with the various contaminations which flow inside the treated water. The bubbles, which do not interact with contaminations, flow up and float at the air-water interface as a result of buoyancy forces. Due to various physical reasons, the partial percentage of the floating bubbles that do not react with contaminations have an average finite life time which results in their explosion into the surrounding air and/or into the treated water. Another component of bubbles that dissolves in the water releases the encapsulated free radicals into the treated water. These mechanisms produce secondary reduction mechanism with liquid hydrogen peroxide.

In a further embodiment of the present invention, the water reservoir is fully or at least partially closed. In a further embodiment of the present invention, the water reservoir is subjected to a high intrinsic internal pressure along the air-water interface as a result of the ejected gas phase of the radicalized/excited gas. This is also in accordance with Henry's Law regarding equilibrium between liquid and gas phase concentrations of any particular species. In a further embodiment of the present invention, the atmospheric pressure is enhanced by an external pressure applied to the water reservoir. In both previous embodiments, there is enhanced interaction of the oxygen gas radicals that flow above the water with water in the reservoir, which results in another generation mechanism of $H_2O_2$ liquid which further cleans the contaminated water. All said reactants comprising free radicals inside the bubbles and $H_2O_2$ react with the dissolved DPD , thereby modifying the water color in the water container.

It is clear from the experimental results that in all experiments the water color is modified to a darker intensity color, suggesting radicals concentration in a certain percentage in the water container. Furthermore, in the reference anti-symmetric magnetic configuration, the color is darker for each compressed gas flow level. This is particularly relevant relative to the configuration without magnetic field. The experimental results show that the darkest color is achieved for the anti-symmetric magnetic configuration, shown in **Fig. 6A,** for compression flow of F=4 litter/min. The less darker color is achieved at such compression flow with the configuration of the ionization chamber without magnetic field, shown in **Fig. 6B.** This clearly indicates that the highest contrast and hence ionization chamber performance are achieved at compression flow of F=4 litter/min. A quantification of the experimental results was done by converting the modified color intensities into values of arbitrary units using the intensity table shown in **Fig. 7B,** where the experimental values are presented in **Figs. 8A.**

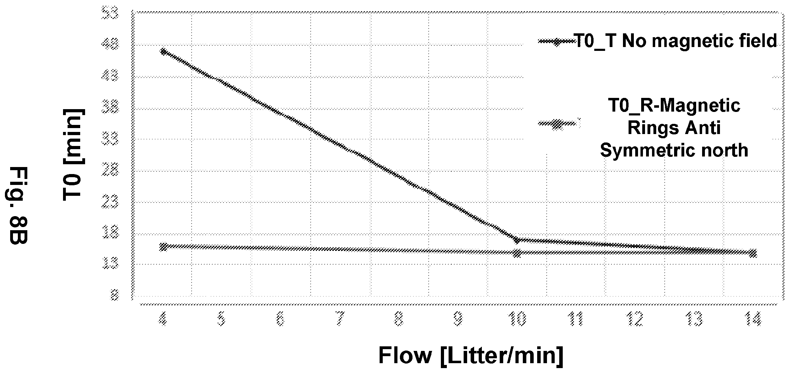

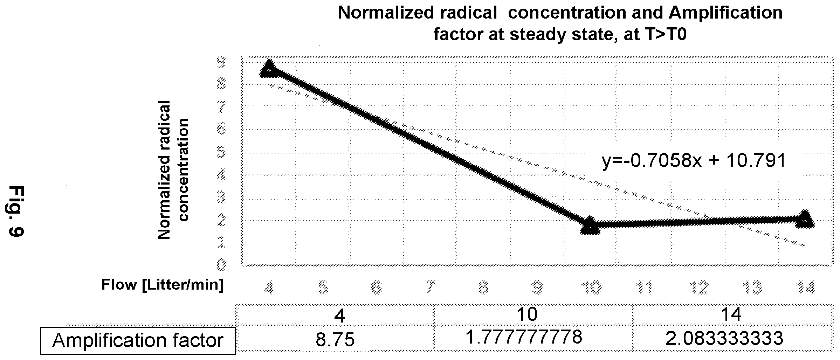

Figs. 8A-B show the full DPD experimental results demonstrated in **Fig. 7.** The experimental results performed for different air compression flows in the ionization chamber presented in **Figs. 1-5,** with the anti-symmetric magnetic configuration, are shown in **Fig. 6A,** and ionization chamber without magnetic field, shown in **Fig. 6B.** In both configurations, the ionization chamber is attached to a small water container. **Fig. 8A** shows the radical concentration, n, at different compression flow values. **Fig. 8B** shows the steady state time stabilization, *T0,* of the system. Experiments were performed inside a small water container, using the DPD experimental gauge device, shown in **Fig. 7A.** Experiments were performed for ionization chamber with magnetic rings in anti-symmetric reference configuration, shown in **Fig. 6A,** and for the ionization chamber without magnetic field shown in **Fig. 6B.** For the configuration of ionization without magnetic field, the concentration grows linearly with the measured increase of compression flow values, as demonstrated by the liner fit grow trend in **Fig. 8A.** The steady state time sharply decreases with the measured increased compression flow, as shown in **Fig. 8B.** These results were expected for the cylindrical ionization chamber which was geometrically designed to effectively operate at high compression flows such as the ones tested in the corresponding experiments. For the ionization chamber with the anti-symmetric magnetic field configuration, the maximum results were achieved for compression flow values of F=4 litter/min, and degraded linearly with compression flow as predicted by the linear fit drop trend, shown in **Fig. 8B.** From both Figs. 8A-B graphs, it is clear that a highest dynamic response with the lowest stabilization time, *T0,* with maximum concentration of radicals, **n,** were achieved for ionization with the anti-symmetric magnetic field at compression flow of F=4 litter/min. Particularly, in that configuration and compression flow, the stabilization time, $T0_m$, is almost a 1/3 of the corresponding stabilization time without magnetic field, $T0_0$, where the radicals concentration is between 8-9 time greater, i.e. $T0_m \sim T0_0/3$ where $n_m \sim n_0/9$. The radicals concentration trend versus the air gas compression flow is shown in **Fig 9.**

In further experiments, we compared the dynamic performances of the ionization chamber for different configurations of magnetic rings, as presented in **Figs. 6 A, C-E.** These configurations represent some exemplary optional embodiments of the ionization chamber system. Experiments were performed at compression rate, **F,** of 4-14 litter/min. The magnetic field configurations in **Figs. 6 A, C-D,** comprise two magnetic sites located around the cylindrical ionization chamber main longitudinal axis, close to its bottom and top ends and adjacent to its air inlet and outlet, respectively. The magnetic field configuration in **Fig. 6E** comprises three magnetic sites, with one site additional to the two previous magnetic sites, which is located at the center of the cylindrical ionization chamber. We measured the stabilization time, **T0,** for each configuration which correlates with the ionization chamber dynamic response. The experimental results for the anti-symmetric configurations, presented in **Fig. 6A** and Figs. 6D-E, show improvement pronounced in a lower stabilization time over all the measured compression rate of 4-14 litter/min, with respect to the symmetric configuration in **Fig. 6C.** We attribute this improvement to the higher magnetic fluxes generally generated by anti-symmetric magnetic field configurations, in which each pair of magnetic rings, in all magnetic sites, is positioned in opposite magnetic polarities, with an identical magnetic orientation inside a certain magnetic configuration.

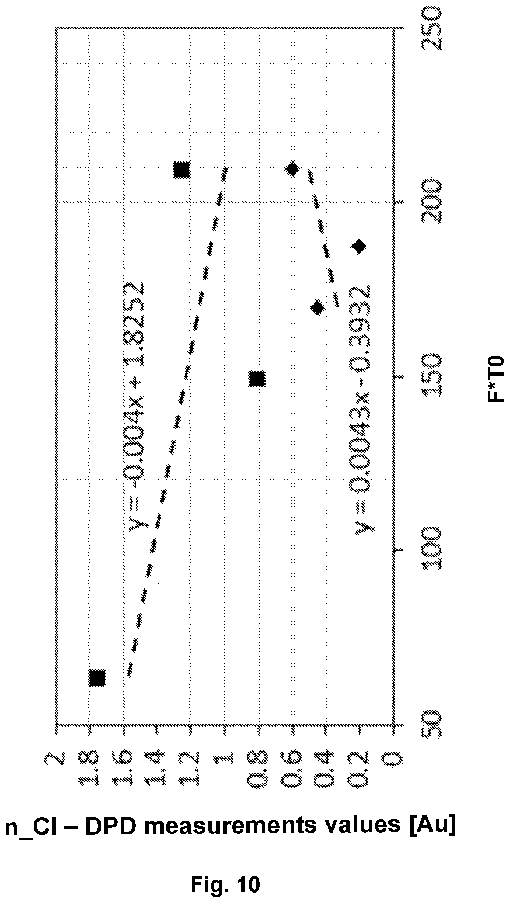

**Fig. 9** shows a graph of the normalized radicals concentration measurement results performed at steady state in different compression flow values. Measurements were performed for ionization chamber with magnetic rings in anti-symmetric reference configuration, shown in **Fig. 6A,** and were normalized to ionization chamber with no magnetic field, shown in **Fig. 6B.** From the graph results, which is also shown the previous graph, it is clear that the optimum of the normalized concentration trend reaches a maximum of $n_m \sim 8-9 n_0$ times the corresponding concentration of the chamber without magnetic field, $n_0$, at compression flow of F=4 litter/min and drop sharply to a factor of $n_m \sim 2 n_0$. This result suggests that in the current magnetic anti-symmetric configuration, the magnetic field is highly effective around air compression value of F=4 litter/min and drops its efficiency with the increase compression magnitude values. To characterize well the dynamic response of the tested ionization chamber in the current preferred embodiment of the present invention, we have plotted the normalized measured DPD concentration values versus parameter, x, in **Fig. 10,** which is equal to the compression air flow by the stabilization time T0, i.e., x=F*T0. The unit of this parameter is **[Litter]** and measures the amount of air which is required to be compressed in order to reach a steady state with a certain concentration. This parameter is characteristic of the ionization chamber dynamic efficiency. We note that measurements performed for ionization chamber with magnetic rings in anti-symmetric reference configuration, shown in **Fig. 6A,** and were normalized to ionization chamber with no magnetic field, shown in **Fig. 6B.**

**Fig. 10** shows graphs of the radicals concentration experimental results versus compression air flow multiplied by the stabilization time **T0** for different compression flow, **F.** Measurements were performed for ionization chamber with magnetic rings in anti-symmetric reference configuration, shown in **Fig. 6A** and ionization chamber configuration without magnetic field, shown in **Fig. 6B.** From these results it appears that for ionization chamber with magnetic rings in an anti-symmetric reference configuration, the ionization chamber dynamic efficiency for $x_m$=60 litter at F=4 litter/min, is 3 times higher with respect to same parameter ionization chamber configuration without magnetic field with corresponding

value of $x_0$=**170 litter.** The concentration of the reference anti-symmetric configuration is $n_m$~8-9 times higher than that of the chamber without magnetic field. We found that this ionization camber has a superior dynamic efficiency, **x,** at compression flow values between **F=4-10 litter/min,** and a superior concentration of radicals, **n,** between **F=4-14 litter/min.** The graphs of the concentration of radicals $n_0$, and of stabilization time, **T0,** versus the compression flow versus the dynamic efficiency parameter, **x=F*TO** shown in Figs.8A-B and Fig. 10, are considered as most important characteristics of the ionization chamber. We use these results as benchmark trends for ionization chamber optimization without the magnetic field combined with high or low compression flows as required by the ionization chamber.

The previous experiments were performed for a small container with a size of **4 litter.** Hence we aim at achieving a parameter value that does not depend on the container volume and diameter and generates an accurate characteristic of the ionization chamber. Accordingly, we have modelled an approximated equation for the average radicals flux density, $\Phi$.

$$1.1 \; (\Phi_m/\; \Phi_0) \sim (n_m/\; n_0\;)*(T0_0/\; T0_m)\;,$$

where the radicals flux with and without a magnetic field is linearly modelled as a multiplication of the radical density fluxes, $\Phi m,\; \Phi_0,$ with the corresponding stabilization times, $Tm_0,\; T0_0,$ as follows:

$$1.2 \; n_m \sim \Phi_m*Tm_0/V, \; \text{and} \; n_0 \sim \Phi_0*T0_0/V,$$

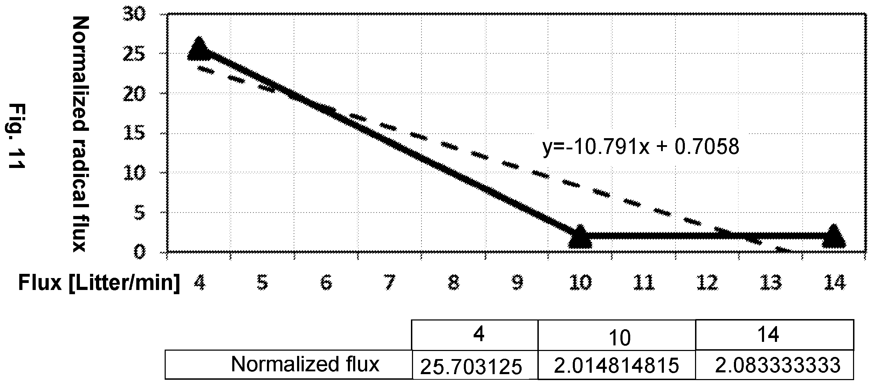

where $V$ is the container volume, $N_m, n_m$ and $N_0, n_0$ are the total number of radicals and their related concentrations, with and without magnetic fields, which are also related as follows: $n_m = N_m/V, n_0 = N_0/V$. **Fig. 11** shows a graph of the normalized calculated average radicals flux density for different compression flow values. Measurements were performed for ionization chamber with magnetic rings in anti-symmetric reference configuration, shown in **Fig. 6A,** and normalized to ionization chamber with no magnetic field, shown in **Fig. 6B.** The results were normalized to calculated average radicals flux density and reached a maximum flux density of, $\Phi_m \sim 25\; \Phi_0,$ times the corresponding concentration of the chamber without magnetic field, $\Phi_0,$ at compression flow of **F=4 litter/min.** Flux density drops sharply to a factor of $\Phi_m \sim 2\Phi_0.$ The value predicted by this model is significantly higher than the measured one as shown for radicals concentration presented in the graph in **Fig. 8A.** Despite the fact that the linear approximation is not accurate, it gives some approximation for the actual contribution and impact of the magnetic field on enhancement of the ionization process, calculated for the anti-symmetric configuration in **Fig. 6A,** and with respect to the configuration with no magnetic field shown in **Fig. 6B.**

## Claims

1. A water purification system (100) comprising:

   a cylindrical chamber (2) comprising inlet and outlet for flowing incoming and outgoing air into said chamber (2) and out of said chamber (2) and into a water-containing tank;
   at least one elongated UV radiation lamp (14) located at center of said cylindrical chamber (2);
   at least one pair of magnetic rings (15a, 15b, 15c);
   an air compressor (8), air pipes (8a) for introducing said air gas into said chamber and output air pipe (2a) for introducing said modified air gas into said water-containing tank;
   a prefilter attached to said air compressor (8) and located before inlet of said chamber (2); and
   a skeleton (13) configured for occupying center volume of said chamber (2) from top to bottom around central longitudinal axis of said chamber (2), said skeleton (13) comprising inner space for accommodating said at least one UV radiation lamp (14) and at least one pair of holding elements (10a, 10b, 10c) for holding said at least one pair of magnetic rings (15a, 15b, 15c) around said at least one UV radiation lamp (14), wherein inner diameter of bases of (17b, 17c) of said skeleton (13) is smaller than inner diameter of a housing sleeve (16), said housing sleeve (16) is connected at opposite ends to top (11) and bottom covers (12), said top (11) and bottom (12) covers are provided each with respective central holes (11a, 12a), and wherein every pair of magnetic rings (15e, 15f) generates a local magnetic field upon placing said pairs of magnetic rings (15e, 15f) on said holding elements (10a, 10b, 10c) of said skeleton (13), said local magnetic field does not overlap or at least minimally interfere with a local magnetic field generated by a neighbor pair of magnetic rings (15e, 15f),
   wherein said purification system (100) comprises concentric configuration to minimally perturb profile and distribution of said incoming and outgoing air, said at least one pair of magnetic rings (15a, 15b, 15c) are positioned in parallel relative each other and configured to induce maximal concentric magnetic flux field on molecules of said flowing incoming and outgoing air.

2. The water purification system (100) according to claim 1, wherein said at least one UV radiation lamp (14) comprises two lamps with two wavelength ranges of 180-195 [nm] and 240-280 [nm].

3. The water purification system (100) according to claim 1, wherein said chamber (2) is made of a conductive material coated with chemically inert material.

4. The water purification system (100) according to claim 1, wherein said chamber (2) further comprises external sleeve and top and bottom covers (11, 12) mechanically attached to top and bottom sides of said external sleeve and close top and bottom ends of said chamber (2).

5. The water purification system (100) according to claim 1, further comprising a plurality of gas flow meters (5), said gas flow meters (5) are mounted inside or outside a box encapsulating said chamber (2).

6. The water purification system (100) according to claim 1, further comprising remote control unit for controlling operational values versus specified values of said system (100), said unit is configured to switch between on and off operating states of said system, mechanically or electronically, and monitor voltage, electrical current, power supply and related devices of said system (100).

7. The water purification system according to claim 6, wherein said devices are selected from said at least one UV lamp (14), a fan (1) for expelling heat generated in said chamber (2) out of said system (100) and electronic air flow meter (5) for monitoring incoming and outgoing air into and out of said chamber (2) within said system (100).

8. The water purification system (100) according to claim 1, further comprising a venturi pipe (2a) attached to said outlet of said chamber for transporting radicalized/excited and ambient air into treated water reservoir.

9. The water purification system (100) according to claim 1, further comprising a water container or water reservoir in fluid communication with said chamber (2).

10. The water purification system (100) according to claim 1, wherein said chamber (2) has a cylindrical geometrical shape with said housing sleeve (16) and housing frame with a corresponding cylindrical geometrical shape.

11. The water purification system (100) according to claim 1, comprising three pairs of magnetic rings (15a, 15b, 15c) arranged in identical polarity configuration at top and bottom ends, center of and around main central longitudinal axis of said chamber (2), wherein each one of said pairs of magnetic rings (15a, 15b, 15c) comprises one ring with negative polarity and second ring with positive polarity, said polarity configuration is anti-symmetric configuration, said rings are mechanically held by said holding elements (10a, 10b, 10c).

12. The water purification system (100) according to claim 11, wherein said magnetic rings generate magnetic field strength in the range of $10^{-3}$ to $10^6$ gauss, said range is sufficient to induce high magnetic flux in said chamber (2) and radicalize incoming ambient air.

13. The water purification system (100) according to claim 1, wherein said skeleton (13) comprises outer longitudinal bars extending from top to bottom of said skeleton around inner space for accommodating said at least one UV radiation lamp (14), and holding elements (10a, 10b, 10c) extending inwardly from said outer bars and comprising recesses for holding said at least one pair of magnetic rings (15a, 15b, 15c) around said at least one UV radiation lamp, said outer bars and holding elements forming a single solid unit of said skeleton.

14. The water purification system (100) according to claim 1, further comprising prefiltering apparatus for cleaning ambient incoming air from impurities and contaminations before injecting it into said chamber (2) and a diffuser connected to outlet of said chamber for diffusing radicalized air into a water reservoir.

**Patentansprüche**

1. Wasserreinigungssystem (100), umfassend:

> eine zylindrische Kammer (2), die einen Einlass und einen Auslass umfasst, um Zu- und Abluft in die Kammer (2) und aus der Kammer (2) und in einen Wasser enthaltenden Tank strömen zu lassen;
> mindestens eine längliche UV-Strahlungslampe (14), die sich in der Mitte der zylindrischen Kammer (2) befindet;
> mindestens ein Paar Magnetringe (15a, 15b, 15c);
> einen Luftkompressor (8), Luftrohre (8a) zum Einleiten des Luftgases in die Kammer und ein Abluftrohr (2a) zum Einführen des modifizierten Luftgases in den Wasser enthaltenden Tank;
> einen Vorfilter, der an dem Luftkompressor (8) angebracht ist und sich vor dem Einlass der Kammer (2) befindet; und
> ein Gerüst (13), das zum Einnehmen eines zentralen Volumens der Kammer (2) von oben nach unten um die zentrale Längsachse der Kammer (2) herum konfiguriert ist, wobei das Gerüst (13) einen Innenraum zum Unterbringen der mindestens einen UV-Strahlungslampe (14) und mindestens ein Paar Halteelemente (10a, 10b, 10c) zum Halten des mindestens einen Paares Magnetringe (15a, 15b, 15c) um die mindestens eine UV-Strahlungslampe (14) herum umfasst, wobei der Innendurchmesser der Sockel von (17b, 17c) des Gerüsts (13) kleiner ist als der Innendurchmesser einer Gehäusehülse (16), wobei die Gehäusehülse (16) an gegenüberliegenden Enden mit einer oberen (11) und unteren Abdeckung (12) verbunden ist, wobei die obere (11) und untere (12) Abdeckung jeweils mit jeweiligen zentralen Löchern (11a, 12a) versehen sind und wobei jedes Paar Magnetringe (15e, 15f) ein lokales Magnetfeld beim Platzieren der Paare Magnetringe (15e, 15f) an den Halteelementen (10a, 10b, 10c) des Gerüsts (13) erzeugt, wobei das lokale Magnetfeld ein von einem benachbarten Paar Magnetringe (15e, 15f) erzeugtes lokales Magnetfeld nicht überlappt oder zumindest minimal stört, wobei das Reinigungssystem (100) eine konzentrische Konfiguration umfasst, um das Profil und die Verteilung der Zu- und Abluft minimal zu stören, wobei das mindestens eine Paar Magnetringe (15a, 15b, 15c) parallel zueinander positioniert ist und so konfiguriert ist, dass sie ein maximales konzentrisches magnetisches Flussfeld auf Molekülen der strömenden Zu- und Abluft induzieren.

2. Wasserreinigungssystem (100) nach Anspruch 1, wobei die mindestens eine UV-Strahlungslampe (14) zwei Lampen mit zwei Wellenlängenbereichen von 180-195 [nm] und 240-280 [nm] umfasst.

3. Wasserreinigungssystem (100) nach Anspruch 1, wobei die Kammer (2) aus einem leitfähigen Material besteht, das mit chemisch inertem Material beschichtet ist.

4. Wasserreinigungssystem (100) nach Anspruch 1, wobei die Kammer (2) ferner eine Außenhülse und eine obere und untere Abdeckung (11, 12) umfasst, die mechanisch an der Ober- und Unterseite der Außenhülse angebracht sind und das obere und untere Ende der Kammer (2) verschließen.

5. Wasserreinigungssystem (100) nach Anspruch 1, ferner umfassend eine Vielzahl von Gasdurchflussmessern (5), wobei die Gasdurchflussmesser (5) innerhalb oder außerhalb eines Kastens montiert sind, der die Kammer (2) umschließt.

6. Wasserreinigungssystem (100) nach Anspruch 1, ferner umfassend eine Fernbedienungseinheit zum Steuern von Betriebswerten im Vergleich zu spezifizierten Werten des Systems (100), wobei die Einheit so konfiguriert ist, dass sie mechanisch oder elektronisch zwischen Ein- und Aus-Betriebszuständen des Systems umschaltet und die Spannung, den elektrischen Strom, die Stromversorgung und zugehörige Geräte des Systems (100) überwacht.

7. Wasserreinigungssystem nach Anspruch 6, wobei die Geräte ausgewählt sind aus der mindestens einen UV-Lampe (14), einem Ventilator (1) zum Ausstoßen von in der Kammer (2) erzeugter Wärme aus dem System (100) und elektronischem Luftdurchflussmesser (5) zur Überwachung von Zu- und Abluft in die und aus der Kammer (2) innerhalb des Systems (100).

8. Wasserreinigungssystem (100) nach Anspruch 1, ferner umfassend ein Venturi-Rohr (2a), das an dem Auslass der Kammer angebracht ist, um radikalisierte/angeregte Luft und Umgebungsluft in einen Vorratsbehälter für behandeltes Wasser zu transportieren.

9. Wasserreinigungssystem (100) nach Anspruch 1, ferner umfassend einen Wasserbehälter oder Wasservorratsbehälter in Fluidverbindung mit der Kammer (2).

10. Wasserreinigungssystem (100) nach Anspruch 1, wobei die Kammer (2) eine zylindrische geometrische Form aufweist, wobei die Gehäusehülse (16) und der Gehäuserahmen eine entsprechende zylindrische geometrische Form aufweisen.

11. Wasserreinigungssystem (100) nach Anspruch 1, umfassend drei Paare Magnetringe (15a, 15b, 15c), die in identischer Polaritätskonfiguration am oberen und unteren Ende, in der Mitte der zentralen Längsachse der Kammer (2) und um diese herum angeordnet sind, wobei jedes der Paare Magnetringe (15a, 15b, 15c) einen Ring mit Minuspolarität und einen zweiten Ring mit Pluspolarität umfasst, wobei die Polaritätskonfiguration eine antisymmetrische Konfiguration ist, wobei die Ringe mechanisch durch die Halteelemente (10a, 10b, 10c) gehalten werden.

12. Wasserreinigungssystem (100) nach Anspruch 11, wobei die Magnetringe eine magnetische Feldstärke im Bereich von $10^{-3}$ bis $10^6$ Gauss erzeugen, wobei dieser Bereich ausreicht, um einen hohen magnetischen Fluss in der Kammer (2) zu induzieren und einströmende Umgebungsluft zu radikalisieren.

13. Wasserreinigungssystem (100) nach Anspruch 1, wobei das Gerüst (13) äußere Längsstäbe, die sich von der Oberseite bis zur Unterseite des Gerüsts um den Innenraum herum zum Unterbringen der mindestens einen UV-Strahlungslampe (14) erstrecken, und Halteelemente (10a, 10b, 10c) umfasst, die sich von den äußeren Stäben nach innen erstrecken und Aussparungen umfassen, um das mindestens eine Paar Magnetringe (15a, 15b, 15c) um die mindestens eine UV-Strahlungslampe herum zu halten, wobei die äußeren Stäbe und Halteelemente eine einzelne feste Einheit des Gerüsts bilden.

14. Wasserreinigungssystem (100) nach Anspruch 1, ferner umfassend eine Vorfiltervorrichtung zum Reinigen von einströmender Umgebungsluft von Verunreinigungen und Kontaminationen, bevor sie in die Kammer (2) eingeblasen wird, und einen Diffusor, der mit dem Auslass der Kammer verbunden ist, um radikalisierte Luft in ein Wasserreservoir zu diffundieren.

**Revendications**

1. Système de purification d'eau (100) comprenant :

une chambre cylindrique (2) comprenant une entrée et une sortie pour faire circuler l'air entrant et sortant dans ladite chambre (2) et hors de ladite chambre (2) et dans un réservoir contenant de l'eau ;
au moins une lampe à rayonnement UV allongée (14) située au centre de ladite chambre cylindrique (2) ;
au moins une paire d'anneaux magnétiques (15a, 15b, 15c) ;
un compresseur d'air (8), des tuyaux d'air (8a) destinés à introduire ledit air gazeux dans ladite chambre et un tuyau d'air de sortie (2a) destiné à introduire ledit air gazeux modifié dans ledit réservoir contenant
de l'eau ;
un préfiltre fixé audit compresseur d'air (8) et situé avant l'entrée de ladite chambre (2) ; et
une structure (13) conçue pour occuper le volume central de ladite chambre (2) de haut en bas autour de l'axe longitudinal central de ladite chambre (2), ladite structure (13) comprenant un espace interne destiné à recevoir ladite au moins une lampe à rayonnement UV (14) et au moins une paire d'éléments de maintien (10a, 10b, 10c) destinés à maintenir ladite au moins une paire d'anneaux magnétiques (15a, 15b, 15c) autour de ladite au moins une lampe à rayonnement UV (14), ledit diamètre interne des bases (17b, 17c) de ladite structure (13) étant plus petit que le diamètre interne d'un manchon (16) de boîtier, ledit manchon (16) de boîtier étant relié au niveau des extrémités opposées aux couvercles supérieur (11) et inférieur (12), lesdits couvercles supérieur (11) et inférieur (12) étant chacun dotés de trous centraux respectifs (11a, 12a), et chaque paire d'anneaux magnétiques (15e, 15f) générant un champ magnétique local lors de la mise en place desdites paires d'anneaux magnétiques (15e, 15f) sur lesdits éléments de maintien (10a, 10b, 10c) de ladite structure (13), ledit champ magnétique local ne chevauchant pas ou n'interférant pas au moins de manière minime avec un champ magnétique local généré par une paire voisine d'anneaux magnétiques (15e, 15f), ledit système de purification (100) comprenant une configuration concentrique pour perturber de manière minime le profil et la distribution dudit air entrant et sortant, ladite au moins une paire d'anneaux magnétiques (15a, 15b, 15c) étant positionnés parallèlement l'un par rapport à l'autre et conçus pour induire un champ de flux magnétique concentrique sur des molécules dudit écoulement d'air entrant et sortant.

2. Système de purification d'eau (100) selon la revendication 1, ladite au moins une lampe à rayonnement UV (14) comprenant deux lampes avec deux plages de longueurs d'onde de 180-195 [nm] et de 240-280 [nm].

3. Système de purification d'eau (100) selon la revendication 1, ladite chambre (2) étant constituée d'un matériau conducteur revêtu d'un matériau chimiquement inerte.

4. Système de purification d'eau (100) selon la revendication 1, ladite chambre (2) comprenant en outre un manchon extérieur et des couvercles supérieur et inférieur (11, 12) étant fixés mécaniquement aux côtés supérieur et inférieur dudit manchon extérieur et fermant les extrémités supérieure et inférieure de ladite chambre (2).

5. Système de purification d'eau (100) selon la revendication 1, comprenant en outre une pluralité de débitmètres de gaz (5), lesdits débitmètres de gaz (5) sont montés à l'intérieur ou à l'extérieur d'un boîtier encapsulant ladite chambre (2).

6. Système de purification d'eau (100) selon la revendication 1, comprenant en outre une unité de commande à distance destinée à commander des valeurs fonctionnelles par rapport aux valeurs spécifiées dudit système (100), ladite unité étant conçue pour commuter entre les états de fonctionnement activé et désactivé dudit système, mécaniquement ou électroniquement et surveiller la tension, le courant électrique, l'alimentation électrique et les dispositifs associés dudit système (100).

**7.** Système de purification d'eau selon la revendication 6, lesdits dispositifs étant sélectionnés parmi ladite au moins une lampe UV (14), un ventilateur (1) destiné à expulser la chaleur générée dans ladite chambre (2) hors dudit système (100) et un débitmètre d'air électronique (5) destiné à surveiller l'air entrant et sortant de ladite chambre (2) à l'intérieur dudit système (100).

**8.** Système de purification d'eau (100) selon la revendication 1, comprenant en outre un tuyau venturi (2a) fixé à ladite sortie de ladite chambre destiné à transporter l'air radicalisé/excité et ambiant dans le réservoir d'eau traitée.

**9.** Système de purification d'eau (100) selon la revendication 1, comprenant en outre un récipient d'eau ou un réservoir d'eau en communication fluidique avec ladite chambre (2).

**10.** Système de purification d'eau (100) selon la revendication 1, ladite chambre (2) comportant une forme géométrique cylindrique avec ledit manchon (16) de boîtier et ledit cadre de boîtier comportant une forme géométrique cylindrique correspondante.

**11.** Système de purification d'eau (100) selon la revendication 1, comprenant trois paires d'anneaux magnétiques (15a, 15b, 15c) agencés selon une configuration de polarité identique au niveau des extrémités supérieure et inférieure, au centre et autour de l'axe longitudinal central principal de ladite chambre (2), chacune desdites paires d'anneaux magnétiques (15a, 15b, 15c) comprenant un anneau à polarité négative et un second anneau à polarité positive, ladite configuration de polarité étant une configuration antisymétrique, lesdits anneaux étant maintenus mécaniquement par lesdits éléments de maintien (10a, 10b, 10c).

**12.** Système de purification d'eau (100) selon la revendication 11, lesdits anneaux magnétiques générant une intensité de champ magnétique dans la plage de $10^{-3}$ à $10^6$ gauss, ladite plage étant suffisante pour induire un flux magnétique élevé dans ladite chambre (2) et radicaliser l'air ambiant entrant.

**13.** Système de purification d'eau (100) selon la revendication 1, ladite structure (13) comprenant des barres longitudinales externes s'étendant de haut en bas de ladite structure autour de l'espace interne pour recevoir ladite au moins une lampe à rayonnement UV (14), et des éléments de maintien (10a, 10b, 10c) s'étendant vers l'intérieur à partir desdites barres externes et comprenant des évidements destinés à maintenir ladite au moins une paire d'anneaux magnétiques (15a, 15b, 15c) autour de ladite au moins une lampe à rayonnement UV, lesdites barres externes et lesdits éléments de maintien formant une seule unité solide de ladite structure.

**14.** Système de purification d'eau (100) selon la revendication 1, comprenant en outre un appareil de préfiltration destiné à nettoyer l'air entrant ambiant des impuretés et contaminations avant de l'injecter dans ladite chambre (2) et un diffuseur raccordé à la sortie de ladite chambre destiné à diffuser de l'air radicalisé dans un réservoir d'eau.

**Fig. 1**

**Fig. 2**

**Fig. 3**

**Fig. 4B**

**Fig. 4A**

**Fig. 5A**

**Fig. 5B**

**Fig. 5C**

**Fig. 5D**

Fig. 6E

Fig. 6D

Fig. 6C

Fig. 6B

Fig. 6A

Fig. 7B

Fig. 7A

EP 3 735 397 B1

|  | 4 | 10 | 14 |
|---|---|---|---|
| ● Magnetic rings Anti Symetric North | 1.75 | 0.8 | 1.25 |
| ◇ No magnetic Filed | 0.2 | 0.45 | 0.6 |

Fig. 8A

$y = -0.0586x + 1.8132$

$y = 0.0401x + 0.0421$

Fig. 8B

Fig. 9

Fig. 10

Fig. 11

| Normalized flux | 25.703125 | 2.0148814815 | 2.0833333333 |
| --- | --- | --- | --- |
| | 4 | 10 | 14 |

y=-10.791x + 0.7058

Normalized radical flux

Flux [Litter/min]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4655933 A, Johnson
- US 9321655 B, Kolstad
- US 2007062883 A, William